# EUROPEAN PATENT SPECIFICATION

(11) **EP 1 617 811 B1**
(45) Date of publication and mention of the grant of the patent: **27.02.2008**
(21) Application number: 04760188.5
(22) Date of filing: 23.04.2004
(51) Int. Cl.: A61K 8/30, A61K 8/18

(54) **STRIPED LIQUID PERSONAL CLEANSING COMPOSITIONS CONTAINING A CLEANSING PHASE AND A SEPARATE BENEFIT PHASE COMPRISING A WATER IN OIL EMULSION**
GESTREIFTE FLÜSSIGE KÖRPERREINIGUNGSZUSAMMENSETZUNGEN MIT EINER REINIGUNGSPHASE UND EINER SEPARATEN VORTEILHAFTEN PHASE MIT EINER WASSER-IN-ÖL-EMULSION
COMPOSITIONS NETTOYANTES LIQUIDES D'HYGIENE PERSONNELLE PRESENTANT DES RAYURES ET CONTENANT UNE PHASE NETTOYANTE ET UNE PHASE BENEFIQUE DISTINCTE COMPORTANT UNE EMULSION HUILEUSE

(30) Priority: 01.05.2003 US 467065 P
(43) Date of publication of application: 25.01.2006
(73) Proprietor: THE PROCTER & GAMBLE COMPANY, Cincinnati, Ohio 45202 (US)
(72) Inventor: WEI, Karl, Shiqing, Mason, OH 45040 (US); THOMAS, Cheyne, Pohlman, Independence, Kentucky 41051 (US); SINE, Mark, Richard, New Richmond, OH 45157 (US)
(74) Representative: L'Huillier, Florent Charles
(86) International application number: PCT/IB2004/051592
(87) International publication number: WO 2004/096162

(56) References cited:
- WO-A-01/70926
- WO-A-96/02229
- WO-A-97/17938
- WO-A-03/055456
- DE-A1- 19 650 952
- US-B1- 6 174 845
- US-B1- 6 306 806
- US-B1- 6 506 391

## Description

### FIELD OF THE INVENTION

The present invention relates to striped liquid personal cleansing compositions comprising a cleansing phase and a separate benefit phase comprising a water in oil emulsion wherein the two phases are packaged in physical contact while remaining stable for long periods of time.

### BACKGROUND OF THE INVENTION

Personal cleansing compositions that purport to provide skin-conditioning benefits are known. Many of these compositions are aqueous systems comprising an emulsified conditioning oil or other similar materials in combination with a lathering surfactant. Although many of these products can provide both conditioning and cleansing benefits, there are often trade-offs associated with their use. For instance, it is often difficult to formulate a product that deposits a sufficient amount of skin conditioning agents on skin during use. In order to combat emulsification of the skin conditioning agents by the cleansing surfactant, large amounts of the skin conditioning agent are often added to the compositions. Unfortunately, raising the level of skin conditioning agent in order to achieve increased deposition can negatively affect product lather performance and stability.

One attempt at providing conditioning and cleansing benefits from a personal cleansing product while maintaining stability has been the use of dual-chamber packaging. These packages comprise separate cleansing compositions and conditioning compositions, and allow for the codispensing of the two in a single or dual stream. The separate conditioning and cleansing compositions thus remain physically separate and stable during prolonged storage and just prior to application, but then mix during or after dispensing to provide conditioning and cleansing benefits from a physically stable system. Although such dual-chamber delivery systems provide improved conditioning benefits versus of conventional systems, it is often difficult to achieve consistent and uniform performance because of the uneven dispensing ratio between the cleansing phase and the conditioning phase from these dual-chamber packages. Additionally, these packaging systems add considerable cost to the finished product.

Striped personal cleansing compositions are also known in the art. However, these compositions do not contain a cleansing phase and a benefit phase and thus stability has not been an issue for these products.

Accordingly, the need still remains for stable personal cleansing compositions that provide both cleansing and improved skin conditioning benefits. It has now been found that striped personal cleansing compositions comprising two phases in physical contact that remain stable for long periods of time can be formulated.

These striped personal cleansing compositions of the present invention comprise cleansing and benefit phases that are packaged in physical contact yet remain stable. These compositions provide improved deposition of conditioning agents on skin.

The compositions of the present invention further provide superior cosmetics via the striped appearance and improved skin feel during and after application. It has been found that such compositions can be formulated with sufficiently high levels of benefit agents without compromising product lather performance and stability. The superior lather performance of these compositions can be demonstrated via the lather volume method described herein.

It has also been found that the striped personal cleansing compositions herein can be formulated with selected skin active agents that provide improved chronic skin benefits to the skin. These compositions comprise a cleansing phase containing a cleansing surfactant and at least one additional benefit phase containing a skin active agent, wherein the cleansing and active phases are packaged in physical contact while remaining stable for long periods of time.

### SUMMARY OF THE INVENTION

The present invention is directed to a striped personal cleansing composition and to a method of delivering skin conditioning benefits to the skin or hair comprising first stripe comprising a cleansing phase comprising a surfactant and water, and at least one additional stripe comprising a separate benefit phase comprising a water in oil emulsion as described in claims 1 and 12.

### DETAILED DESCRIPTION

The striped personal cleansing compositions of the present invention comprise a first stripe comprising a cleansing phase, and at least one separate additional stripe comprising a benefit phase. The benefit phase comprises a water in oil emulsion. These and other essential limitations of the compositions and methods of the present invention, as well as many of the optional ingredients suitable for use herein, are described in detail hereinafter.

By the term "stripe" as used herein, is meant that the cleansing phase and the benefit phase herein occupy separate but distinct physical spaces inside the package in which they are stored, but are in direct contact with one another (i.e., they are not separated by a barrier and they are not emulsified or mixed to any significant degree). In one preferred embodiment of the present invention, the cleansing phase and the benefit phase are present within the container as distinct layers or "stripes". The stripes may be relatively uniform and even across the dimension of the package. Alternatively, the layers may be uneven, i.e. wavy, or may be nonuniform in dimension. The stripes do not need to necessarily extend across the entire dimension of the package. The "stripe' can be various geometric shapes, various different colors or include glitter or pearlescence.

The term "ambient conditions" as used herein, refers to surrounding conditions at one (1) atmosphere of pressure, 50% relative humidity, and 25°C.

The term "stable" as used herein, unless otherwise specified, refers to compositions that maintain at least two "separate" phases when sitting in physical contact at ambient conditions for a period of at least about 180 days. By "separate" is meant that there is substantially no mixing of the phases, observable to the naked eye, prior to dispensing of the composition.

The term "personal cleansing composition" as used herein, refers to compositions intended for topical application to the skin or hair.

The phrase "substantially free of" as used herein, means that the composition comprises less than about 3%, preferably less than about 1%, more preferably less than about 0.5%, even more preferably less than about 0.25%, and most preferably less than about 0.1% of the stated ingredient.

All percentages, parts and ratios as used herein are by weight of the total composition, unless otherwise specified. All such weights as they pertain to listed ingredients are based on the active level and, therefore, do not include solvents or by-products that may be included in commercially available materials, unless otherwise specified.

All cited references are incorporated herein by reference in their entireties. Citation of any reference is not an admission regarding any determination as to its availability as prior art to the claimed invention.

The personal cleansing compositions and methods of the present invention can comprise, consist of, or consist essentially of, the essential elements and limitations of the invention described herein, as well as any additional or optional ingredients, components, or limitations described herein or otherwise useful in personal cleansing compositions intended for topical application to the hair or skin.

### Product Form

The personal cleansing compositions of the present invention are typically in the form of a liquid. The term "liquid" as used herein means that the composition is generally flowable to some degree. "Liquids", therefore, can include liquid, semi-liquid, cream, lotion or gel compositions intended for topical application to skin. These compositions typically exhibit a viscosity of equal to or greater than 3kg/ms (3,000 cps), but less than 1000kg/ms (100,000 cps). These compositions contain a cleansing phase and a benefit phase, both of which are described in greater detail hereinafter.

All of the product forms contemplated for purposes of defining the compositions and methods of the present invention are rinse-off formulations, by which is meant the product is applied topically to the skin or hair and then subsequently (i.e., within minutes) rinsed away with water, or otherwise wiped off using a substrate or other suitable removal means.

### Cleansing Phase

The personal cleansing compositions of the present invention comprise an aqueous cleansing phase that contains a surfactant suitable for application to the skin or hair. Suitable surfactants for use herein include any known or otherwise effective cleansing surfactant which are suitable for application to the skin, and which are otherwise compatible with the other essential ingredients in the aqueous cleansing phase of the compositions. These cleansing surfactants include anionic, nonionic, cationic, zwitterionic or amphoteric surfactants, or combinations thereof. Other suitable surfactants are described in McCutcheon's, Emulsifiers and Detergents, 1989 Annual, published by M. C. Publishing Co., and in U.S. Patent 3,929,678.

The aqueous cleansing phase of the personal care compositions preferably comprises a cleansing surfactant at concentrations ranging from 3% to 60%, more preferably from 4% to 30%, even more preferably from 5% to 25%, by weight of the aqueous cleansing phase. The preferred pH range of the cleansing phase is from 5 to 8.

The aqueous cleansing phase of the personal care compositions preferably produces a Total Lather Volume of at least 350 ml, preferably greater than 400ml, even more preferably greater than 600ml, as described in the Lathering Volume Test. The aqueous cleansing phase of the personal care compositions preferably produces a Flash Lather Volume of at least 150 ml, preferably greater than 200ml, most preferably greater than 300ml as described in the Lathering Volume Test.

Anionic surfactants suitable for use in the cleansing phase include alkyl and alkyl ether sulfates. These materials have the respective formula ROSO₃M and RO(C₂H₄O)ₓSO₃M, wherein R is alkyl or alkenyl of from about 8 to about 24 carbon atoms, x is 1 to 10, and M is a water-soluble cation such as ammonium, sodium, potassium and triethanolamine. The alkyl ether sulfates are typically made as condensation products of ethylene oxide and monohydric alcohols having from about 8 to about 24 carbon atoms. Preferably, R has from about 10 to about 18 carbon atoms in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with about 1 to about 10, preferably from about 3 to about 5, and more preferably with about 3, molar proportions of ethylene oxide and the resulting mixture of molecular species having, for example, an average of 3 moles of ethylene oxide per mole of alcohol, is sulfated and neutralized.

Other suitable anionic surfactants include water-soluble salts of the organic, sulfuric acid reaction products of the general formula [R¹-SO₃-M], wherein R¹ is chosen from the group consisting of a straight or branched chain, saturated aliphatic hydrocarbon radical having from about 8 to about 24, preferably about 10 to about 18, carbon atoms; and M is a cation. Suitable examples are the salts of an organic sulfuric acid reaction product of a hydrocarbon of the methane series, including iso-, neo-, ineso-, and n-paraffins, having about 8 to about 24 carbon atoms, preferably about 10 to about 18 carbon atoms and a sulfonating agent, e.g., SO₃, H₂SO₄, oleum, obtained according to known sulfonation methods, including bleaching and hydrolysis. Preferred are alkali metal and ammonium sulfonated C₁₀₋₁₈ n-paraffins.

Preferred anionic surfactants for use in the cleansing phase include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium tridecyl benzene sulfonate, sodium dodecyl benzene sulfonate, and combinations thereof.

Amphoteric surfactants suitable for use in the cleansing phase include those that are broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent 2,658,072, N-higher alkyl aspartic acids such as those produced according to the teaching of U.S. Patent 2,438,091, and the products described in U.S. Patent 2,528,378.

Zwitterionic surfactants suitable for use in the cleansing phase include those that are broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. Such suitable zwitterionic surfactants can be represented by the formula: 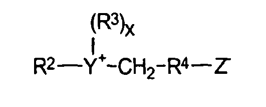 wherein R² contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; R³ is an alkyl or monohydroxyalkyl group containing about I to about 3 carbon atoms; X is 1 when Y is a sulfur atom, and 2 when Y is a nitrogen or phosphorus atom; R⁴ is an alkylene or hydroxyalkylene of from about 1 to about 4 carbon atoms and Z is a radical selected from the group consisting of carboxylate, sulfonate, sulfate, phosphonate, and phosphate groups.

Other zwitterionic surfactants suitable for use in the cleansing phase include betaines, including high alkyl betaines such as coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis- (2-hydroxyethyl) carboxymethyl betaine, stearyl bis- (2-hydroxypropyl) carboxymethyl betaine, oleyl dimethyl gamma-carboxypropyl betaine, and lauryl bis-(2-hydroxypropyl)alphacarboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like; amidobetaines and amidosulfobetaines, wherein the RCONH(CH₂)₃ radical is attached to the nitrogen atom of the betaine are also useful in this invention.

### Characteristics of Cleansing Phase Preferred For Stability

### Lamellar Structurant

The compositions of the present invention preferably comprise 0.1% to 10% by wt. of a structurant agent in the cleansing phase which functions in the compositions to form a lamellar phase. It is believed the lamellar phase enhances the interfacial stability between the cleansing phase and the benefit phase.

Suitable structurant include fatty acids or ester derivatives thereof, a fatty alcohol, or trihydroxystearin, polycare 133. More preferably the structurant is selected from lauric acid or trihydroxystearin.

In an additional embodiment of the present invention the surfactant compositions for use in the cleansing phase exhibit Non-Newtonian shear thinning behavior (herein referred to as free flowing compositions). These cleansing compositions comprising water, at least one anionic surfactant, an electrolyte and at least one alkanolamide. It has been found that by employing a cleansing phase exhibiting Non-Newtonian shear thinning behavior, the stability of the resulting personal cleansing composition may be increased.

The alkanolamide if present has the general structure of: 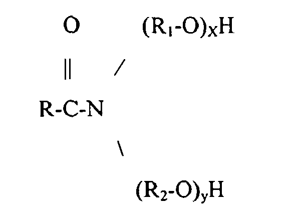 wherein R is C₈ to C₂₄ or preferably in some embodiments C₈ to C₂₂ or in other embodiments C₈ to C₁₈ saturated or unsaturated straight chain or branched aliphatic group, R₁ and R₂ are the same or different C₂-C₄ straight chain or branched aliphatic group, x = 0 to 10; y = 1 - 10 and wherein the sum of x and y is less than or equal to 10.

The amount of alkanolamide when present in the composition is 0.1% to 10% by weight, and in some embodiments is preferably 2% to 5% by weight. Some preferred alkanolamides include Cocamide MEA (Coco monethanolamide) and Cocamide MIPA (Coco monoisopropranotamide). A co-surfactant from the classes of nonionic surfactant, amphoteric and/or zwitterionic surfactant or cationic surfactant may be optionally incorporated.

The electrolyte, if used, can be added per se to the composition or it can be formed in situ xia the counter-ions included in one of the raw materials. The electrolyte preferably includes an anion comprising phosphate, chloride, sulfate or citrate and a cation comprising sodium, ammonium, potassium, magnesium or mixtures thereof. Some preferred electrolytes are sodium or ammonium chloride or sodium or ammonium sulfate.

The electrolyte should be present in an amount, which facilitates formation of the free flowing composition. Generally, this amount is from 0.1% by weight to 15% by weight, preferably from 1% to 6% by weight of the cleansing phase, but may be varied if required.

### Optional Ingredients for use in the Cleansing Phase

Other suitable optional ingredients which may be employed in the cleansing phase include humectants and solutes. A variety of humectants and solutes can be employed and can be present at a level of from 0.1 % to 50 %, preferably from 0.5 % to 35 %, and more preferably from 2 % to 20% of the personal care composition.

Nonionic polyethylene/polypropylene glycol polymers are preferably used as skin conditioning agents. Polymers useful herein that are especially preferred are PEG-2M wherein x equals 2 and n has an average value of about 2,000 (PEG 2-M is also known as Polyox WSR® N-10 from Union Carbide and as PEG-2,000); PEG-5M wherein x equals 2 and n has an average value of about 5,000 (PEG 5-M is also known as Polyox WSR® 35 and Polyox WSR® N-80, both from Union Carbide and as PEG-5,000 and Polyethylene Glycol 200,000); PEG-7M wherein x equals 2 and n has an average value of about 7,000 (PEG 7-M is also known as Polyox WSR® (N-750 from Union Carbide); PEG-9M wherein x equals 2 and n has an average value of about 9,000 (PEG 9-M is also known as Polyox WSR® N-3333 from Union Carbide); PEG-14 M wherein x equals 2 and n has an average value of about 14,000 (PEG 14-M is also known as Polyox WSR-205 and Polyox WSR® N-3000 both from Union Carbide); and PEG-90M wherein x equals 2 and n has an average value of about 90,000 (PEG-90M is also known as Polyox WSR®-301 from Union Carbide.)

The striped personal cleansing compositions of the present invention may additionally comprise an organic cationic deposition polymer in the cleansing phase as a deposition aid for the benefit agents described hereinafter. Concentrations of the cationic deposition polymer preferably range from 0.025% to 3%, more preferably from 0.05% to 2%, even more preferably from 0.1% to 1%, by weight of the cleansing phase composition.

Suitable cationic deposition polymers for use in the striped personal cleansing composition of the present invention contain cationic nitrogen-containing moieties such as quaternary ammonium or cationic protonated amino moieties. The cationic protonated amines can be primary, secondary, or tertiary amines (preferably secondary or tertiary), depending upon the particular species and the selected pH of the personal cleansing composition. The average molecular weight of the cationic deposition polymer is between 5,000 to 10 million, preferably at least 100,000, more preferably at least 200,000, but preferably not more than 2 million, more preferably not more than 1.5 million. The polymers also have a cationic charge density ranging from 0.2 meq/gm to 5 meq/gm, preferably at least 0.4 meq/gm, more preferably at least 0.6 meq/gm., at the pH of intended use of the personal cleansing composition, which pH will generally range from pH 4 to pH 9, preferably between pH 5 and pH 8.

The charge density can be controlled and adjusted in accordance with techniques well known in the art. As used herein the "charge density" of the cationic polymers is defined as the number of cationic sites per polymer gram atomic weight (molecular weight), and can be expressed in terms of meq/gram of cationic charge. In general, adjustment of the proportions of amine or quaternary ammonium moieties in the polymer, as well as pH of the personal cleansing composition in the case of the amines, will affect the charge density.

Any anionic counterions can be use in association with the cationic deposition polymers so long as the polymers remain soluble in water, in the personal cleansing composition, or in a coacervate phase of the personal cleansing composition, and so long as the counterions are physically and chemically compatible with the essential components of the personal cleansing composition or do not otherwise unduly impair product performance, stability or aesthetics. Nonlimiting examples of such counterions include halides (e.g., chlorine, fluorine, bromine, iodine), sulfate and methlylsulfate.

Nonlimiting examples of cationic deposition polymers for use in the personal cleansing composition include polysaccharide polymers, such as cationic cellulose derivatives. Preferred cationic cellulose polymers are the salts of hydroxyethyl cellulose reacted with trimethyl ammonium substituted epoxide, referred to in the industry (CTFA) as Polyquaternium 10 which are available from Amerchol Corp. (Edison, N.J., USA) in their Polymer KG, JR and LR series of polymers with the most preferred being KG-30M.

Other suitable cationic deposition polymers include cationic guar gum derivatives, such as guar hydroxypropyltrimonium chloride, specific examples of which include the Jaguar series (preferably Jaguar C-17) commercially available from Rhodia Inc., and N-Hance polymer series commercially available from Aqualon.

Other suitable cationic deposition polymers include synthetic cationic polymers. The cationic polymers suitable for use in the cleansing composition herein is water soluble or dispersible, non crosslinked, cationic polymers having a cationic charge density of from 4 meq/gm to 7 meq/gm, preferably from 4 meq/gm to 6 meq/gm, more preferably from 4.2 meq/gm to 5.5 meq/gm. The select polymers also must have an average molecular weight of from 1,000 to 1 million, preferably from 10,000 to 500,000, more preferably from 75,000 to 250,000.

The concentration of the cationic polymer in the cleansing composition ranges 0.025% to 5%, preferably from 0.1% to 3%, more preferably from 0.2% to 1%, by weight of the composition.

A non-limiting example of a commercially available synthetic cationic polymer for use in the cleansing compositions is polymethyacrylamidopropyl trimonium chloride, available under the trade name Polycare 133, from Rhodia, Cranberry, N.J., U.S.A.

The cationic polymers herein are either soluble in the cleansing phase, or preferably are soluble in a complex coacervate phase in the striped personal cleansing composition formed by the cationic deposition polymer and the anionic surfactant component described hereinbefore. Complex coacervates of the cationic deposition polymer can also be formed with other charged materials in the personal cleansing composition.

Coacervate formation is dependent upon a variety of criteria such as molecular weight, component concentration, and ratio of interacting ionic components, ionic strength (including, modification of ionic strength, for example, by addition of salts), charge density of the cationic and anionic components, pH, and temperature. Coacervate systems and the effect of these parameters have been described, for example, by J. Caelles, et al., "Anionic and Cationic Compounds in Mixed Systems", Cosmetics & Toiletries, Vol. 106, April 1991, pp 49-54, C. J. van Oss, "Coacervation, Complex-Coacervation and Flocculation", J. Dispersion Science and Technology, Vol. 9 (5,6), 1988-89, pp 561-573, and D. J. Burgess, "Practical Analysis of Complex Coacervate Systems", J. of Colloid anti Interface Science, Vol. 140, No. 1, November 1990, pp 227-238.

It is believed to be particularly advantageous for the cationic deposition polymer to be present in the personal cleansing composition in a coacervate phase, or to form a coacervate phase upon application or rinsing of the cleansing composition to or from the skin. Complex coacervates are believed to more readily deposit on the skin, which results in improved deposition of the benefit materials. Thus, in general, it is preferred that the cationic deposition polymer exists in the personal cleansing composition as a coacervate phase or form a coacervate phase upon dilution. If not already a coacervate in the personal cleansing composition, the cationic deposition polymer will preferably exist in a complex coacervate form in the cleansing composition upon dilution with water.

Techniques for analysis of formation of complex coacervates are known in the art. For example, centrifugation analyses of the personal cleansing compositions, at any chosen stage of dilution, can be utilized to identify whether a coacervate phase has formed.

### Benefit Phase (Water in Oil Emulsion)

The benefit phase of the present invention comprises a water in oil emulsion comprising an oil, an emulsifier, water and preferably a density modifier. The oil phase is the continuous phase and the water phase is the discontinuous or "internal" phase.

### Oils

The benefit phase of the present invention typically comprises from 10% to 99% of oil, more preferably 20 to 95% oil, even more preferably from 50 to 90% oil and most preferably from 60% to 80%.

In general the higher the level of oil employed in the water in oil emulsion the more stable the personal cleansing composition employing the water in oil emulsion will be. Oils suitable for use herein include any natural and synthetic materials with an overall solubility parameter less than about 12.5 (cal/cm³)^{0.5}, preferably less than about 11.5 (cal/cm³)^{0.5}. Solubility parameters for the oils described herein are determined by methods well known in the chemical arts for establishing the relative polar character of a material. A description of solubility parameters and means for determining them are described by C. D. Vaughn, "Solubility Effects in Product, Package, Penetration and Preservation" 103 Cosmetics and Toiletries 47-69, October 1988; and C. D. Vaughn, "Using Solubility Parameters in Cosmetics Formulation", 36 J. Soc. Cosmetic Chemists 319-333, September/October, 1988.

The benefit agent for use in the benefit phase of the composition has a Vaughan Solubility Parameter (VSP) of from 5 to 10, preferably from 6 to less than 10, more preferably from 6 to 9. Non-limiting examples of benefit agents having VSP values ranging from 5 to 10 include the following:

### VAUGHAN SOLUBILITY PARAMETERS*

| | |
|---|---|
| Cyclomethicone | 5.92 |
| Squalene | 6.03 |
| Petrolatum | 7.33 |
| Isopropyl Palmitate | 7.78 |
| Isopropyl Myristate | 8.02 |
| Castor Oil | 8.90 |
| Cholesterol | 9.55 |

**·** As reported in Solubility, Effects in Product, Package, Penetration and Preservation, C. D. Vaughan, Cosmetics and Toiletries, Vol. 103, October 1988.

By "overall solubility parameter" is meant that it is possible to use oils with higher solubility parameters than 12.5 (cal/cm ³)^{0.5} if they are blended with other oils to reduce the overall solubility parameter of the oil mixture to less than about 12.5 (cal/cm³)^{0.5}. For example, a small portion of diethylene glycol (sol par = 13.61) could be blended with lanolin oil (sol par = 7.3) and a cosolublizing agent to create a mixture that has a solubility parameter of less than 12.5(cal/cm³)^{0.5}.

Suitable for use herein oils include but are not limited, to hydrocarbon oils and waxes, silicones, fatty acid derivatives, cholesterol, cholesterol derivatives, diglycerides, triglycerides, vegetable oils, vegetable oil derivatives, acetoglyceride esters, alkyl esters, alkenyl esters, lanolin and its derivatives, wax esters, beeswax derivatives, sterols and phospholipids, and combinations thereof.

Non-limiting examples of hydrocarbon oils and waxes suitable for use herein include petrolatum, mineral oil, micro-crystalline waxes, polyalkenes, paraffins, cerasin, ozokerite, polyethylene, perhydrosqualene, poly alpha olefins, hydrogenated polyisobutenes and combinations thereof.

Non-limiting examples of silicone oils suitable for use herein include dimethicone copolyol, dimethylpolysiloxane, diethylpolysiloxane, mixed C1-C30 alkyl polysiloxanes, phenyl dimethicone, dimethiconol, and combinations thereof. Preferred are non-volatile silicones selected from dimethicone, dimethiconol, mixed C1-C30 alkyl polysiloxane, and combinations thereof. Nonlimiting examples of silicone oils useful herein are described in U.S. Patent No. 5,011,681 (Ciotti et al.).

Non-limiting examples of diglycerides and triglycerides suitable for use herein include castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil and sesame oil, vegetable oils, sunflower seed oil, and vegetable oil derivatives; coconut oil and derivatized coconut oil, cottonseed oil and derivatized cottonseed oil, jojoba oil, cocoa butter, and combinations thereof. In addition any of the above oils that have been partially or fully hydrogenated are also suitable.

Non-limiting examples of acetoglyceride esters suitable for use herein include acetylated monoglycerides.

Non-limiting examples of alkyl esters suitable for use herein include isopropyl esters of fatty acids and long chain esters of long chain fatty acids, e.g. SEFA (sucrose esters of fatty acids). Lauryl pyrolidone carboxylic acid, pentaerthritol esters, aromatic mono, di or triesters, cetyl ricinoleate, non-limiting examples of which include isopropyl palmitate, isopropyl myristate, cetyl riconoleate and stearyl riconoleate. Other examples are: hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof.

Non-limiting examples of alkenyl esters suitable for use herein include oleyl myristate, oleyl stearate, oleyl oleate, and combinations thereof.

Non-limiting examples of lanolin and lanolin derivatives suitable for use herein include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol riconoleate, hydroxylated lanolin, hydrogenated lanolin and combinations thereof.

Still other suitable oils include milk triglycerides (e.g., hydroxylated milk glyceride) and polyol fatty acid polyesters.

Still other suitable oils include wax esters, non-limiting examples of which include beeswax and beeswax derivatives, spermaceti, myristyl myristate, stearyl stearate, and combinations thereof. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and combinations thereof.

### Low HLB Emulsifier

The water-in-oil emulsion of the present invention also includes 0.1% to 20% of a low HLB emulsifier, more preferably from 0.1% to 10%, still more preferably from 0.5% to 9%, of one or more low HLB emulsifier. The low HLB emulsifier may function as an emulsifier

Preferred low HLB emulsifiers are those having an HLB of from about 1 to about 10, more preferably from 1 to about 8. Suitable low HLB emulsifiers are those selected from saturated C₁₄ to C₃₀ fatty alcohols, saturated C₁₆ to C₃₀ fatty alcohols containing from about 1 to about 5 moles of ethylene oxide, saturated C₁₆ to C₃₀ diols, saturated C₁₆ to C₃₀ monoglycerol ethers, saturated C₁₆ to C₃₀ hydroxy fatty acids, C₁₄ to C₃₀ hydroxylated and nonhydroxylated saturated fatty acids, C₁₄ to C₃₀ saturated ethoxylated fatty acids, amines and alcohols containing from about 1 to about 5 moles of ethylene oxide diols, C₁₄ to C₃₀ saturated glyceryl mono esters with a monoglyceride content of at least 40%, C₁₄ to C₃₀ saturated polyglycerol esters having from about 1 to about 3 alkyl group and from about 2 to about 3 saturated glycerol units, C₁₄ to C₃₀ glyceryl mono ethers, C₁₄ to C₃₀ sorbitan mono/diesters, C₁₄ to C₃₀ saturated ethoxylated sorbitan mono/diesters with about 1 to about 5 moles of ethylene oxide, C₁₄ to C₃₀ saturated methyl glucoside esters, C₁₄ to C₃₀ saturated sucrose mono/diesters, C₁₄ to C₃₀ saturated ethoxylated methyl glucoside esters with about 1 to about 5 moles of ethylene oxide, C₁₄ to C₃₀ saturated polyglucosides having an average of between 1 to 2 glucose units and mixtures thereof, having a melting point of at least about 45°C.

The low HLB emulsifiers of the present invention are selected from stearic acid, palmitic acid, stearyl alcohol, cetyl alcohol, behenyl alcohol, stearic acid, palmitic acid, the polyethylene glycol ether of stearyl alcohol having an average of 1 to 5 ethylene oxide units, the polyethylene glycol ether of cetyl alcohol having an average of 1 to 5 ethylene oxide units, and mixtures thereof. More preferred low HLB emulsifiers of the present invention are selected from stearyl alcohol, cetyl alcohol, behenyl alcohol, the polyethylene glycol ether of stearyl alcohol having an average of about 2 ethylene oxide units (steareth-2), the polyethylene glycol ether of cetyl alcohol having an average of about 2 ethylene oxide units, and mixtures thereof. Even more preferred low HLB emulsifiers are selected from stearic acid, palmitic acid, stearyl alcohol, cetyl alcohol, behenyl alcohol, steareth-2, and mixtures thereof.

### Density Modifiers

To further improve stability under stress conditions such as high temperature and vibration, it is preferable to adjust the densities of the separate phases such that they are substantially equal. To achieve this, low density microspheres are added to the cleansing phase of the striped composition. The low density microspheres employed to reduce the overall density of the cleansing phase are particles having a density lower than 0.7 g/cm³, preferably less than 0.2 g/cm³, more preferably less than 0.1 g/cm³, most preferably less than 0.05 g/cm³. The low density microspheres generally have a diameter less than 200 µm, preferably less than 100 µm, most preferably less than 40 µm. Preferably, the density difference between the cleansing phase and the benefit phase is less than 0.15 g/cm³, more preferably, the density difference is less than 0.10 g/cm³, even more preferably, the density difference is less than 0.05g/cm³, most preferably, the density difference is less than 0.01 g/cm³.

The microspheres are produced from any appropriate inorganic or organic material, compatible with a use on the skin, that is, nonirritating and nontoxic.

Expanded microspheres made of thermoplastic material are known, and may be obtained, for example, according to the processes described in Patents and Patent Applications EP-56219, EP-348372, EP-486080, EP-320473, EP-112807 and U.S. Pat. No. 3,615,972.

These microspheres may be produced from any nontoxic and non-irritant thermoplastic materials. Polymers or copolymers of acrylonitrile or of vinylidene chloride may be used, for example. It is possible to use, for example, a copolymer containing, by weight, from 0 to 60% of units derived from vinylidene chloride, from 20 to 90% of units derived from acrylonitrile and from 0 to 50% of units derived from an acrylic or styrene monomer, the sum of the percentages (by weight) being equal to 100. The acrylic monomer is, for example, a methyl or ethyl acrylate or methacrylate. The styrene monomer is, for example, alpha-methylstyrene or styrene. These microspheres can be in the dry or hydrated state.

The internal cavity of expanded hollow microspheres contains a gas, which can be a hydrocarbon such as isobutane or isopentane or alternatively air. Among hollow microspheres which can be used, special mention may be made of those marketed under the brand name EXPANCEL® (thermoplastic expandable microspheres) by the Akzo Nobel Company, especially those of DE (dry state) or WE (hydrated state) grade. Examples include: Expancel ® 091 DE 40 d30; Expancel ® 091 DE 80 d30; Expancel ® 051 DE 40 d60; Expancel ® 091 WE 40 d24; Expancel ® 053 DE 40 d20.

Representative microspheres derived from an inorganic material, include, for instance, "Qcel ® Hollow Microspheres" and "EXTENDOSPHERES^{™} Ceramic Hollow Spheres", both available from the PQ Corporation. Examples are: Qcel® 300; Qcel ® 6019; Qcel ® 6042S.

Just as low density microspheres can be added to the cleansing phase of the present invention to improve vibrational stability, high density materials can be added to the benefit phase to increase its density having the same impact on stability.

### Aqueous Phase

The benefit phase of the present invention typically comprises from 1% to 90% of an aqueous phase. The aqueous phase comprises a fluid selected from the group consisting of water, mono- and polyhydric alcohols (glycerin, propylene glycol, ethanol, isopropanol, etc.).

### Optional Ingredients

The personal cleansing compositions of the present invention may further comprise other optional ingredients that may modify the physical, chemical, cosmetic or aesthetic characteristics of the compositions or serve as additional "active" components when deposited on the skin. The compositions may also further comprise optional inert ingredients. Many such optional ingredients are known for use in personal care compositions, and may also be used in the personal cleansing compositions herein, provided that such optional materials are compatible with the essential materials described herein, or do not otherwise unduly impair product performance.

Such optional ingredients are most typically those materials approved for use in cosmetics and that are described in reference books such as the CTFA Cosmetic Ingredient Handbook, Second Edition, The Cosmetic, Toiletries, and Fragrance Association, Inc. 1988, 1992. These optional materials can be used in any aspect of the compositions of the present invention, including either of the active or cleansing phases as described herein.

Optional ingredients for use in the cleansing phase of the compositions of the present invention can include any benefit phase material as described herein that is also compatible with the selected ingredients in the cleansing phase. Likewise, optional ingredients for use in the benefit phase of the compositions of the present invention can include any cleansing phase material described herein that is also compatible with the selected ingredients in the benefit phase.

Other optional ingredients for use in either phase of the composition, preferably the benefit phase, include silicone elastomer powders and fluids to provide any of a variety of product benefits, including improved product stability, application cosmetics, emolliency, conditioning, and so forth. The concentration of the silicone elastomers in the composition preferably ranges from 0.1% to 20%, more preferably from 0.5% to 10%, by weight of the composition. In this context, the weight percentages are based upon the weight of the silicone elastomers material itself, excluding any silicone-containing fluid that typically accompanies such silicone elastomers materials in the formulation process. The silicone elastomers suitable for optional use herein include emulsifying and non-emulsifying silicone elastomers, non-limiting examples of which are described in U.S.S.N. 09/613,266 (assigned to The Procter & Gamble Company).

The separate benefit phase of the striped liquid personal cleansing compositions may optionally comprise the following skin benefit ingredients for enhanced delivery of these water in oil emulsion materials on skin. Non limiting examples of these optional ingredients include vitamins and derivatives thereof (e.g., ascorbic acid, vitamin E, tocopheryl acetate, and the like); sunscreens; thickening agents (e.g., polyol alkoxy ester, available as Crothix from Croda); preservatives for maintaining the anti microbial integrity of the cleansing compositions; anti-acne medicaments (resorcinol, salicylic acid, and the like); antioxidants; skin soothing and healing agents such as aloe vera extract, allantoin and the like; chelators and sequestrants; and agents suitable for aesthetic purposes such as fragrances, essential oils, skin sensates, pigments, pearlescent agents (e.g., mica and titanium dioxide), lakes, colorings, and the like (e.g., clove oil, menthol, camphor, eucalyptus oil, and eugenol).

### Method of Use

The striped personal cleansing compositions of the present invention are preferably applied topically to the desired area of the skin or hair in an amount sufficient to provide effective delivery of the skin conditioning agent to the applied surface, or to otherwise provide effective skin conditioning benefits. The compositions can be applied directly to the skin or indirectly via the use of a cleansing puff, washcloth, sponge or other implement. The compositions are preferably diluted with water prior to, during, or after topical application, and then subsequently rinsed or wiped off of the applied surface, preferably rinsed off of the applied surface using water or a water-insoluble substrate in combination with water.

### Method of Manufacture

The personal cleansing compositions of the present invention may be prepared by any known or otherwise effective technique, suitable for making and formulating the desired striped product form. It is effective to combine toothpaste-tube filling technology with a spinning stage design. Additionally, the present invention can be prepared by the method and apparatus as disclosed in U.S. patent 6,213,166. The method and apparatus allows two or more compositions to be filled with a spiral configuration into a single container. The method requires that at least two nozzles be employed to fill the container. The container is placed on a static mixer and spun as the composition is introduced into the container.

Alternatively, it is especially effective to combine at least two phases by first placing the separate compositions in separate storage tanks having a pump and a hose attached. The phases are then pumped in predetermined amounts into a single combining section. Next, the phases are moved from the combining sections into the blending sections and the phases are mixed in the blending section such that the single resulting product exhibits a distinct pattern of the phases. The next step involves pumping the product that was mixed in the blending section via a hose into a single nozzle, then placing the nozzle into a container and filing the container with the resulting product. Specific non-limiting examples of such methods as they are applied to specific embodiments of the present invention are described in the following examples.

If the personal cleansing compositions contain stripes of varying colors it may be desirable to package these compositions in a transparent package such that the consumer can view the pattern through the package. Because of the viscosity of the subject compositions it may also be desirable to include instructions to the consumer to store the package upside down, on its cap to facilitate dispensing.

### Analytical Methods

### Lather Volume

Lather volume of a striped liquid personal cleansing composition is measured using a graduated cylinder and a tumbling apparatus. A 1,000 ml graduated cylinder is chosen which is marked in 10 ml increments and has a height of 14.5 inches at the 1,000 ml mark from the inside of its base (for example, Pyrex No. 2982). Distilled water (100 grams at 23°C) is added to the graduated cylinder. The cylinder is clamped in a rotating device, which clamps the cylinder with an axis of rotation that transects the center of the graduated cylinder. One gram of the total personal cleansing composition (0.5g of the cleansing phase and 0.5g of the benefit phase when measuring the total product, or I g of the cleansing phase when the measuring the cleansing phase only) is added into the graduated cylinder and the cylinder is capped. The cylinder is rotated at a rate of 10 revolutions in about 20 seconds, and stopped in a vertical position to complete the first rotation sequence. A timer is set to allow 30 seconds for the lather thus generated to drain. After 30 seconds of such drainage, the first lather volume is measured to the nearest 10 ml mark by recording the lather height in ml up from the base (including any water that has drained to the bottom on top of which the lather is floating).

If the top surface of the lather is uneven, the lowest height at which it is possible to see halfway across the graduated cylinder is the first lather volume (ml). If the lather is so coarse that a single or only a few foam cells ("bubbles") reach across the entire cylinder, the height at which at least 10 foam cells are required to fill the space is the first lather volume, also in ml up from the base. Foam cells larger than one inch in any dimension, no matter where they occur, are designated as unfilled air instead of lather. Foam that collects on the top of the graduated cylinder but does not drain is also incorporated in the measurement if the foam on the top is in its own continuous layer, by adding the ml of foam collected there using a ruler to measure thickness of the layer, to the ml of foam measured up from the base. The maximum foam height is 1,000 ml (even if the total foam height exceeds the 1,000 ml mark on the graduated cylinder). One minute after the first rotation is completed, a second rotation sequence is commenced which is identical in speed and duration to the first rotation sequence. The second lather volume is recorded in the same manner as the first, after the same 30 seconds of drainage time. A third sequence is completed and the third lather volume is measured in the same manner, with the same pause between each for drainage and taking the measurement.

The lather result after each sequence is added together and the Total Lather Volume determined as the sum of the three measurements, in ml. The Flash Lather Volume is the result after the first rotation sequence only, in ml, i.e., the first lather volume. Compositions according to the present invention perform significantly better in this test than similar compositions in conventional emulsion form.

### Viscosity of the Liquid Personal Cleansing Composition

The Wells-Brookfield Cone/Plate Model DV-II+ Viscometer can be used to determine the viscosity of the liquid personal cleansing compositions herein. The determination is performed at 25°C with the 2.4cm⁰ cone measuring system with a gap of 0.013mm between the two small pins on the respective cone and plate. The measurement is performed by injecting 0.5ml of the sample, to be analyzed, between the cone and plate and, then, rotating the cone at a set speed of 1 rpm. The resistance to the rotation of the cone produces a torque that is proportional to the shear stress of the liquid sample. The amount of torque is read 2 minutes after loading the sample and computed by the viscometer into absolute centipoise units (mPa*s) based on the geometric constant of the cone, the rate of rotation, and the stress related torque.

### Yield Point of Liquid Personal Cleansing Composition

The Carrimed CSL 100 Controlled Stress Rheometer can be used to determine the yield point of the liquid personal cleansing compositions. For purpose herein, the yield point is the amount of stress required to produce a strain of 1% on the liquid personal cleansing composition. The determination is performed at 77F with the 4cm 2°cone measuring system set with a 51 micron gap. The determination is performed via the programmed application of a shear stress (typically from 0.06 dynes/sq. centimeter to 500 dynes/sq. centimeter) over time interval of 5 minutes. It is this amount of stress that results in a deformation of the sample, a shear stress vs. strain curve can be created. From this curve, the yield point of the liquid personal cleansing composition can be calculated.

### EXAMPLES

The following examples further describe and demonstrate embodiments within the scope of the present invention. The examples are given solely for the purpose of illustration and are not to be construed as limitations of the present invention. All exemplified amounts are concentrations by weight of the total composition, i.e., wt/wt percentages, unless otherwise specified.

Each of the exemplified compositions provides improved deposition or effectiveness of the skin conditioning agents or optional ingredients delivered from each prepared composition. Examples 1-3.

The following examples described in Table 1 are non-limiting examples of the personal cleaning compositions herein.

**Table 1: Cleansing Phase and Benefit phase Compositions**

| | **Example 1** | **Example 2** | **Example 3** |
|---|---|---|---|
| Ingredient | **wt%** | **wt%** | **Wt%** |
| **I. Cleansing Phase Composition** | | | |
| Ammonium Laureth-3 Sulfate | 3.0 | 3.0 | 3.0 |
| Sodium Lauroamphoacetate (Miranol L-32 Ultra from Rhodia) | 16.7 | 16.7 | 16.7 |
| Ammonium Lauryl Sulfate | 1.0 | 1.0 | 1.0 |
| Lauric Acid | 0.9 | 0.9 | 0.9 |
| Trihydroxystearin (Thixcin R) | 2.0 | 2.0 | 2.0 |
| Guar Hydroxypropyltrimonium Chloride (N-Hance 3196 from Aqualon) | 0.17 | 0.75 | 0.75 |
| Guar Hydroxypropyltrimonium Chloride (Jaguar C-17 from Rhodia) | 0.58 | - | - |
| Polyquaterium 10 (UCARE polymer JR-30M from Amerchol) | 0.45 | - | - |
| Polymethacrylamidopropyltrimonium Chloride (Polycare 133 from Rhodia) | - | 0.24 | - |
| Polyquatemium-39 (Merqurt Plus 3300 from Calgon | - | 0.81 | - |
| PEG 90M (Polyox WSR 301 from Union Carbide) | 0.25 | - | - |
| PEG-14M (Polyox WSR N-3000 H from Union Carbide) | 0.45 | 2.45 | 2.45 |
| Linoleamidoprypyl PG-Dimonium Chloride Phosphate Dimethicone (Monasil PLN from Uniqema) | - | 1.0 | 4.0 |
| Glycerin | 1.4 | 4.9 | 4.9 |
| Sodium Chloride | 0.3 | 0.3 | 0.3 |
| Sodium Benzoate | 0.25 | 0.25 | 0.25 |
| Disodium EDTA | 0.13 | 0.13 | 0.13 |
| Glydant | 0.37 | 0.37 | 0.37 |
| Citric Acid | 1.6 | 0.95 | 0.95 |
| Titanium Dioxide | 0.5 | 0.5 | 0.5 |
| Perfume | 0.5 | 0.5 | 0.5 |
| Water | Q.S. | Q.S. | Q.S. |
| | | | |
| **II. Benefit Composition** | | | |
| Petrolatum | 80 | 80 | 80 |
| PEG-30 Dipolyhydroxystearate (Arlacel P135) | 1 | 1 | 1 |
| Water | 19 | 19 | 19 |

The cleansing phase and benefit phase compositions described above can be prepared by conventional formulation and mixing techniques. The cleansing composition 1 can be prepared by first creating the following premixes: citric acid in water premix at 1:3 ratio, Guar polymer premix with Jaguar C-17 and N-Hance 3196 in water at 1:10 ratio, UCARE premix with JR-30M in water at about 1:30 ratio, and Polyox premix with PEG-90M and PEG-14M in Glycerin at about 1:2 ratio. Then, the following ingredients will be added into the main mixing vessel: ammonium lauryl sulfate, ammonium laureth-3 sulfate, citric acid premix, Miranol L-32 ultra, sodium chloride, sodium benzoate, disodium EDTA, lauric acid, Thixcin R, Guar premix, UCARE premix, Polyox Premix, and the rest of water. Then one will heat the vessel with agitation until it reaches 190°F (88°C). Let it mix for about 10 min. Cool the batch with a cold water bath with slow agitation until it reaches 110°F (43°C). Add the following ingredients: Glydant, perfume, Titanium Dioxide. Mix until a homogeneous solution forms.

The cleansing composition 2 can be prepared by first creating the following premixes: citric acid in water premix at 1:3 ratio, Guar polymer premix with N-Hance 3196 in water at 1:10 ratio, and Polyox premix with PEG-14M in Glycerin at about 1:2 ratio. Then, the following ingredients will be added into the main mixing vessel: ammonium lauryl sulfate, ammonium laureth-3 sulfate, citric acid premix, Miranol L-32 ultra, sodium chloride, sodium benzoate, disodium EDTA, lauric acid, Thixcin R, Guar premix, Polyox Premix, Polycare 133, Merquat Plus 3300, Monosil PLN, and the rest of water. Then, the vessel will be heated with agitation until it reaches 190°F (88°C). Let it mix for about 10 min. Next, the batch will be cooled with a cold water bath with slow agitation until it reaches 110°F (43°C). Finally, the following ingredients will be added: Glydant, perfume, Titanium Dioxide and mixed until a homogeneous solution forms.

The cleansing composition 3 can be prepared by first creating the following premixes: citric acid in water premix at 1:3 ratio, Guar polymer premix with N-Hance 3196 in water at 1:10 ratio, and Polyox premix with PEG-14M in Glycerin at about 1:2 ratio. Then, the following ingredients will be added into the main mixing vessel: ammonium lauryl sulfate, ammonium laureth-3 sulfate, citric acid premix, Miranol L-32 ultra, sodium chloride, sodium benzoate, disodium EDTA, lauric acid, Thixcin R, Guar premix, Polyox Premix, Monasil PLN, and the rest of water. Then the vessel will be heated with agitation until it reaches 190°F (88°C). The vessel will be mixed for about 10 min. Next, the batch will be cooled with a cold water bath with slow agitation until it reaches 110°F (43°C). Finally, the following ingredients will be added: Glydant, perfume, Titanium Dioxide and mixed until a homogeneous solution forms.

### Benefit phase

The benefit phase can be prepared by adding Petrolatum into the main mixing vessel. Then, the vessel will be heated to 185°F and add Arlacel P135. Then, slowly add water with agitation. Keep agitating until homogeneous.

The cleansing and benefit phases are packaged into a single container by first placing the separate compositions in separate storage tanks having a pump and a hose attached. The phases are then pumped in predetermined amounts into a single combining section. Next, the phases are moved from the combining sections into the blending sections and the phases are mixed in the blending section such that the single resulting product exhibits a distinct pattern of the phases. The next step involves pumping the product that was mixed in the blending section via a hose into a single nozzle, then placing the nozzle into a container and filing the container with the resulting product. The sample stage spins the bottle during the filling process to create a striped appearance.

### Examples 4-6

The following examples described in Table 2 are non-limiting examples of the personal cleaning compositions herein. Table 2: Cleansing Phase and Benefit phase Compositions

| | **Example 4** | **Example 5** | **Example 6** |
|---|---|---|---|
| **Ingredient** | **wt%** | **wt%** | **wt%** |
| **I. Cleansing Phase Composition** | | | |
| Miracare SLB-365 (from Rhodia) (Sodium Trideceth Sulfate, Sodium Lauramphoacetate, Cocamide MEA) | 47.4 | 47.4 | 47.4 |
| Guar Hydroxypropyltrimonium Chloride ( N-Hance 3196 from Aqualon) | | - | 0.7 |
| PEG 90M (Polyox WSR 301 from Dow Chemical) | - | - | 0.2 |
| Cocamide MEA | 3.0 | - | - |
| Polycare 133 | - | - | 0.4 |
| Lauric Acid | - | 2.0 | 2.0 |
| Sodium Chloride | 3.5 | 3.5 | 3.5 |
| Disodium EDTA | 0.05 | 0.05 | 0.05 |
| Glydant | 0.67 | 0.67 | 0.67 |
| Citric Acid | 0.4 | 0.4 | 0.4 |
| Perfume | 2.0 | 2.0 | 2.0 |
| Water | Q.S. | Q.S. | Q.S. |
| (pH) | (6.0) | (6.0) | (6.0) |
| **II. Benefit phase Composition** | | | |
| Petrolatum | 80 | 80 | 80 |
| PEG-30 Dipolyhydroxystearate (Arlacel P135) | 1 | 1 | 1 |
| Water | 19 | 19 | 19 |

The compositions described above can be prepared by conventional formulation and mixing techniques. The cleansing phase composition can be prepared by first adding citric acid into water at 1:3 ratios to form a citric acid premix. The following ingredients will then be added into the main mixing vessel in the following sequence: water, Miracare SLB-354, sodium chloride, sodium benzoate, Disodium EDTA, glydant. The main mixing vessel will start to be agitated. In a separate mixing vessel, disperse polymers (Polyquaterium 10, Jaguar C-17, or N-Hance 3196) in water at 1:10 ratio will form a polymer premix. The completely dispersed polymer premix will be added into the main mixing vessel with continuous agitation. Polyox WSR 301 will be dispersed in water and then added to the main mixing vessel. Then, the rest of the water and perfume will be added into the batch. The batch will be kept agitating until a homogenous solution forms.

### Benefit phase

The benefit phase can be prepared by adding Petrolatum into the main mixing vessel. Then, the vessel will be heated to 185F and add Arlacel P135. Then, slowly add water with agitation. Keep agitating until homogeneous.

The cleansing and benefit phases are packaged into a single container by first placing the separate compositions in separate storage tanks having a pump and a hose attached. The phases are then pumped in predetermined amounts into a single combining section. Next, the phases are moved from the combining sections into the blending sections and the phases are mixed in the blending section such that the single resulting product exhibits a distinct pattern of the phases. The next step involves pumping the product that was mixed in the blending section via a hose into a single nozzle, then placing the nozzle into a container and filing the container with the resulting product.. The sample stage spins the bottle during filling process to create a striped appearance.

### Examples 7-9

The following examples described in Table 3 are non-limiting examples of the personal cleaning compositions herein. Table 3: Cleansing Phase and Benefit phase Compositions

| | **Example 7** | **Example 8** | **Example 9** |
|---|---|---|---|
| **Ingredient** | **wt%** | **wt%** | **wt%** |
| **I. Cleansing Phase Composition** | | | |
| Miracare SLB-365 (from Rhodia) (Sodium Trideceth Sulfate, Sodium Lauramphoacetate, Cocamide MEA) | 47.4 | 47.4 | 47.4 |
| Sodium Chloride | 3.5 | 3.5 | 3.5 |
| Disodium EDTA | 0.05 | 0.05 | 0.05 |
| Glydant | 0.67 | 0.67 | 0.67 |
| Citric Acid | 0.4 | 0.4 | 0.4 |
| Perfume | 2.0 | 2.0 | 2.0 |
| Water | Q.S. | Q.S. | Q.S. |
| (pH) | (6.0) | (6.0) | (6.0) |
| **II. Benefit phase Composition** | | | |
| Petrolatum | 80 | 80 | 80 |
| PEG-30 Dipolyhydroxystearate (Arlacel P135 | 1 | 1 | 1 |
| Water | 19 | 19 | 19 |

The compositions described above can be prepared by conventional formulation and mixing techniques. The cleansing phase composition can be prepared by first adding citric acid into water at 1:3 ratio to form a citric acid premix. The following ingredients will be added into the main mixing vessel in the following sequence: water, Miracare SLB-354, sodium chloride, sodium benzoate, Disodium EDTA, glydant. The main mixing vessel will start to be agitated. Then, perfume will be added into the batch. The batch will be kept agitating until a homogenous solution forms.

### Benefit phase

The benefit phase can be prepared by adding Petrolatum into the main mixing vessel. Then, the vessel will be heated to 185F and add Arlacel P135. Then, slowly add water with agitation. Keep agitating until homogeneous.

The cleansing and benefit phases are packaged into a single container by first placing the separate compositions in separate storage tanks having a pump and a hose attached. The phases are then pumped in predetermined amounts into a single combining section. Next, the phases are moved from the combining sections into the blending sections and the phases are mixed in the blending section such that the single resulting product exhibits a distinct pattern of the phases. The next step involves pumping the product that was mixed in the blending section via a hose into a single nozzle, then placing the nozzle into a container and filing the container with the resulting product. The sample stage spins the bottle during the filling process to create a striped appearance.

## Claims

1. A striped personal cleansing composition comprising:
(a) a first stripe comprising a cleansing phase comprising a surfactant and water; and
(b) at least one additional stripe comprising a benefit phase comprising a water in oil emulsion;
wherein the cleansing phase and the benefit phase are packaged in physical contact with one another and maintain stability, wherein the cleansing phase additionally comprises a lamellar structurant, **characterised in that**
the cleansing phase comprises:
(i) at least one anionic surfactant;
(ii) at least one electrolyte;
(iii) at least one alkanolamide;
and
wherein the cleansing phase is non-Newtonian shear thinning, and has a viscosity of equal to or greater than 3kg/ms (3000 cps).

2. A striped personal cleansing composition according to any one of the preceding claims, wherein the surfactant comprises from 3% to 60% by weight of the aqueous cleansing phase.

3. A striped personal cleansing composition according to Claim 1, wherein the electrolyte comprises:
i) an anion selected from the group consisting of phosphate, chloride, sulfate, citrate and mixtures thereof, and
ii) a cation selected from the group consisting of sodium, ammonium, potassium, magnesium and mixtures thereof; and wherein the electrolyte is present from 0.1% to 15% by weight of the cleansing phase.

4. A striped personal cleansing composition according to Claim 1 wherein said benefit phase comprises an emulsifier having HLB below 10 and wherein said emulsifier is selected from PEG-30 dipolyhydroxystearate, dimethicone copolyol, and mixtures thereof.

5. A striped personal cleansing composition according to any one of the preceding claims, wherein said benefit phase comprises from 10% to 90% of an oil.

6. A striped personal cleansing composition according to any one of the preceding claims, which comprises a density modifier; preferably wherein the density modifier is a hollow microsphere.

7. A striped personal cleansing composition according to any one of the preceding claims, further comprising a cationic deposition polymer in said cleansing phase; preferably wherein cationic deposition polymer is selected from the group of cationic cellulosic derivatives, cationic guar derivatives, cationic synthetic polymers, and mixtures thereof.

8. A striped personal cleansing composition according to any one of the preceding claims, wherein the lamellar structurant is selected from fatty acids, fatty esters, trihydroxystearin, fatty alcohols, and mixture thereof.

9. A striped personal cleansing composition according to any one of the preceding claims, wherein at least one phase contains a colorant; preferably wherein the cleansing and benefit phases visually form a pattern within the package; more preferably wherein the pattern is selected from the group consisting of striped, marbled, geometric, and mixtures thereof; even more preferably wherein the composition is packaged in a transparent container.

10. A striped personal cleansing composition according to any one of the preceding claims, wherein the composition is packaged in a container with instructions to store said container on the lid

11. A striped personal cleansing composition according to any one of the preceding claims, wherein the composition comprises skin care actives, wherein the skin care actives are selected from the group consisting of vitamins and derivatives thereof; sunscreens; thickening agents; preservatives; anti-acne medicaments; antioxidants; skin soothing and healing; chelators and sequestrants; fragrances, essential oils, skin sensates, pigments, pearlescent agents, lakes, colorings, and mixtures thereof.

12. A method of delivering skin conditioning benefits to the skin or hair, said method comprising the steps of:
a) dispensing an effective amount of a composition according to claim 1 onto an implement selected from the group consisting of a cleansing puff, washcloth, sponge and human hand;
b) topically applying said composition to the skin or hair using said implement; and
c) removing said composition from the skin or hair by rinsing with water.

## Patentansprüche

1. Gestreifte Körperreinigungszusammensetzung, umfassend:
(a) einen ersten Streifen, umfassend eine Reinigungsphase, die ein Tensid und Wasser umfasst; und
(b) mindestens einen zusätzlichen Streifen, umfassend eine Nutzphase, die eine Wasser-in-Öl-Emulsion umfasst;
wobei die Reinigungsphase und die Nutzphase in physischem Kontakt miteinander verpackt sind und Stabilität bewahren, wobei die Reinigungsphase zusätzlich ein Lamellenstrukturmittel umfasst, **dadurch gekennzeichnet, dass** die Reinigungsphase Folgendes umfasst:
(i) mindestens ein anionisches Tensid;
(ii) mindestens einen Elektrolyten;
(iii) mindestens ein Alkanolamid; und
wobei die Reinigungsphase nichtnewtonsch strukturviskos ist und eine Viskosität von gleich oder größer als ungefähr 3 kg/ms (3000 cP) aufweist.

2. Gestreifte Körperreinigungszusammensetzung nach einem der vorstehenden Ansprüche, wobei das Tensid von 3 Gew.-% bis 60 Gew.-% der wässrigen Reinigungsphase ausmacht.

3. Gestreifte Körperreinigungszusammensetzung nach Anspruch 1, wobei der Elektrolyt Folgendes umfasst:
i) ein Anion, ausgewählt aus der Gruppe, bestehend aus Phosphat, Chlorid, Sulfat, Citrat und Mischungen davon, und
ii) ein Kation, ausgewählt aus der Gruppe, bestehend aus Natrium, Ammonium, Kalium, Magnesium und Mischungen davon; und wobei der Elektrolyt von 0,1 Gew.-% bis 15 Gew.-% der Reinigungsphase vorhanden ist.

4. Gestreifte Körperreinigungszusammensetzung nach Anspruch 1, wobei die Nutzphase einen Emulgator mit einem HLB-Wert unter 10 umfasst und wobei der Emulgator aus PEG-30-Dipolyhydroxystearat, Dimethiconcopolyol und Mischungen davon ausgewählt ist.

5. Gestreifte Körperreinigungszusammensetzung nach einem der vorstehenden Ansprüche, wobei die Nutzphase von 10 % bis 90 % ein Öl umfasst.

6. Gestreifte Körperreinigungszusammensetzung nach einem der vorstehenden Ansprüche, die ein Dichtemodifizierungsmittel umfasst, wobei das Dichtemodifizierungsmittel vorzugsweise eine hohle Mikrokugel ist.

7. Gestreifte Körperreinigungszusammensetzung nach einem der vorstehenden Ansprüche, die ferner ein kationisches Abscheidepolymer in der Reinigungsphase umfasst, wobei das kationische Abscheidepolymer vorzugsweise ausgewählt ist aus der Gruppe von kationischen Cellulosederivaten, kationischen Guargummiderivaten, kationischen synthetischen Polymeren und Mischungen davon.

8. Gestreifte Körperreinigungszusammensetzung nach einem der vorstehenden Ansprüche, wobei das Lamellenstrukturmittel aus Fettsäuren, Fettsäureestern, Trihydroxystearin, Fettalkoholen und Mischungen davon ausgewählt ist.

9. Gestreifte Körperreinigungszusammensetzung nach einem der vorstehenden Ansprüche, wobei mindestens eine Phase ein Färbemittel enthält; wobei die Reinigungs- und Nutzphase vorzugsweise visuell ein Muster innerhalb der Verpackung bilden; wobei das Muster mehr bevorzugt ausgewählt ist aus der Gruppe, bestehend aus gestreift, marmoriert, geometrisch und Mischungen davon; wobei die Zusammensetzung noch mehr bevorzugt in einem transparenten Behälter verpackt ist.

10. Gestreifte Körperreinigungszusammensetzung nach einem der vorstehenden Ansprüche, wobei die Zusammensetzung in einem Behälter mit Anweisungen zum Lagern des Behälters auf dem Deckel verpackt ist.

11. Gestreifte Körperreinigungszusammensetzung nach einem der vorstehenden Ansprüche, wobei die Zusammensetzung Hautpflegewirkstoffe umfasst, wobei die Hautpflegewirkstoffe ausgewählt sind aus der Gruppe, bestehend aus Vitaminen und Derivaten davon; Sonnenschutzmitteln; Verdickungsmitteln; Konservierungsstoffen; aknehemmenden Medikamenten; Antioxidationsmitteln; Mitteln zur Linderung und Heilung gereizter Haut; Komplexbildnern und Sequestriermitteln; Düften, etherischen Ölen, Mitteln zur Verbesserung der Hautempfindung, Pigmenten, Perlglanzmitteln, Farblacken, Färbemitteln und Mischungen davon.

12. Verfahren zur Lieferung von Hautkonditionierungsvorteilen für die Haut oder das Haar, wobei das Verfahren die folgenden Schritte umfasst:
a) Abgeben einer wirksamen Menge einer Zusammensetzung nach Anspruch 1 auf eine Vorrichtung, ausgewählt aus der Gruppe, bestehend aus einem Reinigungsbausch, einem Waschlappen, einem Schwamm und einer menschlichen Hand;
b) äußerliches Auftragen der Zusammensetzung auf die Haut oder das Haar mit der Vorrichtung; und
c) Entfernung der Zusammensetzung von der Haut oder dem Haar durch Spülen mit Wasser.

## Revendications

1. Composition pour la toilette en bandes comprenant :
(a) une première bande comprenant une phase de nettoyage comprenant un agent tensioactif et de l'eau ; et
(b) au moins une bande supplémentaire comprenant une phase bénéfique comprenant une émulsion eau dans huile ;
dans laquelle la phase de nettoyage et la phase bénéfique sont conditionnées en contact physique l'une avec l'autre et maintiennent la stabilité, dans laquelle la phase de nettoyage comprend, en outre, un agent structurant lamellaire, **caractérisé en ce que** la phase de nettoyage comprend :
(i) au moins un agent tensioactif anionique ;
(ii) au moins un électrolyte ;
(iii) au moins un alcanolamide ; et
dans laquelle la phase de nettoyage est rhéofluidifiante non newtonienne, et a une viscosité égale ou supérieure à environ n3 kg/ms (3000 cP).

2. Composition pour la toilette en bandes selon l'une quelconque des revendications précédentes, dans laquelle l'agent tensioactif constitue de 3 % à 60 % en poids de la phase de nettoyage aqueuse.

3. Composition pour la toilette en bandes selon la revendication 1, dans laquelle l'électrolyte comprend :
i) un anion choisi dans le groupe constitué de phosphate, chlorure, sulfate, citrate et leurs mélanges, et
ii) un cation choisi dans le groupe constitué de sodium, ammonium, potassium, magnésium et leurs mélanges ; et dans laquelle l'électrolyte est présent de 0,1% à 15 % en poids de la phase de nettoyage.

4. Composition pour la toilette en bandes selon la revendication 1, dans laquelle ladite phase bénéfique comprend un émulsifiant ayant un rapport HLB inférieur à 10 et dans laquelle ledit émulsifiant est choisi dans le groupe constitué de PEG-30 dipolyhydroxystéarate, diméthicone copolyol, et leurs mélanges.

5. Composition pour la toilette en bandes selon l'une quelconque des revendications précédentes, dans laquelle ladite phase bénéfique comprend de 10 % à 90 % d'une huile.

6. Composition pour la toilette en bandes selon l'une quelconque des revendications précédentes, qui comprend un agent modifiant la masse volumique, de préférence dans laquelle l'agent modifiant la masse volumique est une microsphère creuse.

7. Composition pour la toilette en bandes selon l'une quelconque des revendications précédentes, comprenant, en outre, un polymère de dépôt cationique dans ladite phase de nettoyage, de préférence dans laquelle le polymère de dépôt cationique est choisi dans le groupe constitué de dérivés cellulosiques cationiques, dérivés de gomme guar cationiques, polymères synthétiques cationiques, et leurs mélanges.

8. Composition pour la toilette en bandes selon l'une quelconque des revendications précédentes, dans laquelle l'agent structurant lamellaire est choisi parmi des acides gras, des esters gras, la trihydroxystéarine, des alcools gras, et leurs mélanges.

9. Composition pour la toilette en bandes selon l'une quelconque des revendications précédentes, dans laquelle au moins une phase contient un colorant ; de préférence
dans laquelle les phases de nettoyage et bénéfique forment visuellement un motif au sein du conditionnement ; plus préférablement dans laquelle le motif est choisi dans le groupe constitué de motifs en bandes, marbrés, géométriques, et leurs mélanges ; encore plus préférablement dans laquelle la composition est conditionnée dans un conteneur transparent.

10. Composition pour la toilette en bandes selon l'une quelconque des revendications précédentes, dans laquelle la composition est conditionnée dans un conteneur avec un mode d'emploi pour stocker ledit conteneur sur le couvercle.

11. Composition pour la toilette en bandes selon l'une quelconque des revendications précédentes, dans laquelle la composition comprend des principes actifs de soin de la peau, dans laquelle les principes actifs de soin de la peau sont choisis dans le groupe constitué de vitamines et dérivés de celles-ci ; écrans solaires ; agents épaississants ; conservateurs ; médicaments anti-acnéiques ; antioxydants ; agents émollients et de soin ; agents chélatants et séquestrants ; parfums, huiles essentielles, sensates cutanés, pigments, agents perlescents, laques, agents colorants, et leurs mélanges.

12. Procédé de libération d'effets bénéfiques de conditionnement cutané sur la peau ou les cheveux, ledit procédé comprenant les étapes consistant à :
a) distribuer une quantité efficace d'une composition selon la revendication 1, sur un instrument choisi dans le groupe constitué d'un tampon de nettoyage, un gant de toilette, une éponge et une main humaine ;
b) appliquer localement ladite composition sur la peau ou les cheveux à l'aide dudit instrument ; et
c) enlever ladite composition de la peau ou des cheveux par rinçage à l'eau.
